# EUROPEAN PATENT SPECIFICATION

(11) **EP 2 668 852 B1**
(45) Date of publication and mention of the grant of the patent: **16.08.2017**
(21) Application number: 11857185.0
(22) Date of filing: 16.12.2011
(51) Int. Cl.: A23L 27/00, A23L 33/10, A23L 33/165

(54) **COMPOSITION FOR ORAL ADMINISTRATION**
ZUSAMMENSETZUNGEN ZUR ORALEN VERABREICHUNG
COMPOSITION POUR ADMINISTRATION ORALE

(30) Priority: 24.01.2011 JP 2011012245
(43) Date of publication of application: 04.12.2013
(73) Proprietor: FUJIFILM Corporation, Tokyo 106-8620 (JP)
(72) Inventor: TSUJIHATA, Shigetomo, Ashigarakami-gun Kanagawa 258-8577 (JP); HASHIMOTO, Shinichi, Ashigarakami-gun Kanagawa 258-8577 (JP)
(74) Representative: Hoffmann Eitle
(86) International application number: PCT/JP2011/079253
(87) International publication number: WO 2012/101931

(56) References cited:
- WO-A1-2010/143719
- JP-A- 11 196 786
- JP-A- 50 123 854
- JP-A- 61 238 754
- JP-A- H10 114 683
- JP-A- 2002 503 268
- JP-A- 2006 006 251
- JP-A- 2006 008 857
- JP-A- 2006 055 074
- JP-A- 2009 240 297
- JP-A- 2009 263 298
- JP-A- 2010 209 029
- JP-A- 2010 268 707
- DATABASE WPI Week 200842 Thomson Scientific, London, GB; AN 2008-G62517 XP002740380, & JP 2008 094837 A (DAINIPPON PHARM CO LTD) 24 April 2008 (2008-04-24)

## Description

### Technical Field

The present invention relates to a composition for oral administration.

### Background Art

In recent years, food materials having functions are extensively studied with a focus on tertiary functions of foods. Polyphenols, amino acids and peptides are representative examples of such food materials. In a case in which these food materials are processed to be used in foods or supplements, the tastes of these food materials, particularly bitterness and astringency, often cause problems. Many of low-molecular-weight physiologically active substances derived from plants generally have bitterness and astringency, and, therefore, it has been difficult to ingest the required amount of these substances even using food materials having health functionality.

Examples of such materials include iso-α acids such as isohumulone. Iso-α acids, which are extracts from hop, play a role in brewing beer and low-malt beer, and also have various useful physiological functions such as an anti-caries effect, an anti-osteoporosis effect, a growth inhibition effect on *Helicobacter pylori,* or a blood sugar level improving effect. With this background, iso-α acids are expected to be applied to health-conscious foods and drinks such as so-called health foods and functional foods.

For example, Japanese Patent Application National Publication (JP-A) No. 2008-543942 discloses a hop acid formulation having improved bioavailability, the formulation being obtained by adding an inorganic salt of calcium or the like to iso-α acids to form a slurry, and then drying the slurry. However, iso-α acids have a unique bitterness, and, when iso-α acids are used in drinks or foods, inclusion of the required amount of iso-α acids causes strong unpleasant sensation. Therefore, it has been difficult to realize drinks or foods containing a high level of iso-α acid.

In view of these circumstances, some technologies have been proposed with a view to reducing bitterness and astringency.

Reported methods of masking bitterness in foods, pharmaceutical products, and the like having bitterness include (1) a method in which an artificial sweetener such as aspartame, acesulfame potassium, or the like is used (see Japanese Patent Application Laid-Open (JP-A) No. H02-56416 and Japanese Patent No. 4347161); (2) a method in which a bitter component is trapped in cyclodextrin (see JP-A No. H03-236316 and WO2007/066773); (3) a method in which a calcium compound is added to catechin, which is a bitter component (see JP-A No. 2007-289158): or (4) a method in which zinc gluconate or the like is added to a bitter component (see JP-A No. 2006-6251).

JP-A-2009-263298 teaches a pharmaceutical composition comprising diphenhydramine hydrochloride as a bitter-tasting substance, aluminium hydroxide and corn starch as a dispersant.

JP-A-2006-006251 discloses that divalent copper or zinc salts of gluconic acid can be used to mask bitterness.

### SUMMARY OF INVENTION

### Technical Problem

However, the conventional methods described above are still insufficient as a method for masking bitterness. Further, in a case in which a bitter or astringent component such as an iso-α acid is subjected to insolubilization treatment, such as addition of a metal salt for forming a slurry, and used in food or drink, there is a tendency that unpleasant sensation is left in the mouth and graininess is sensed.

As described above, a composition for oral administration in which bitterness or astringency is reduced in order to allow effective ingestion of a bitter or astringent substance has not been obtained.

Further, in a case in which a composition containing an insolubilized bitter or astringent substance is made to have liquid form such as a drink, a problem arises in that the insolubilized material precipitates, thereby deteriorating the outer appearance.

Therefore, an object of the present invention is to provide a composition for oral administration in which bitterness or astringency generated by a bitter or astringent substance is reduced, and which has transparency not impairing the outer appearance when the composition is made to have liquid form, for example, a dispersion liquid.

### Solution to Problem

According to a first aspect, the present invention provides a composition for oral administration comprising:
a water-insolubilized material which does not completely dissolve in water at 25°C obtainable by combining a bitter or astringent substance and a trivalent iron salt; and
a dispersant.

Preferably the bitter or astringent substance includes an organic acid, a hop extract or an iso-α acid.

Preferably the bitter or astringent substance includes a combination of iso-α acid and an α-acid.

Preferably the dispersant is a water-soluble macromolecule and/or a surfactant.

Preferably the dispersant is a water-soluble macromolecule selected from gelatin, gum arabic, modified starch, pullulan and a cellulose derivative.

Preferably the composition contains 0.01 to 100 moles of a divalent or trivalent salt per mole of the bitter or astringent substance.

Preferably the content of the dispersant is from 0.1 times to 100 times the content of the bitter or astringent substance in terms of a mass ratio.

Preferably the composition is in the form of a dispersion liquid including dispersed particles, and the dispersed particles include the water-insolubilized material. In this case, the average particle diameter of the dispersant particles is 300 nm or less.

According to a second aspect, the present invention provides a method for reducing bitterness or astringency of a composition that includes a bitter or astringent substance, the method including combining the bitter or astringent substance with a dispersant and a trivalent salt of iron.

### Advantageous Effects of Invention

According to the present invention, a composition for oral administration in which bitterness or astringency generated by a bitter or astringent substance is reduced, and which has transparency not impairing the outer appearance when the composition is made to have liquid form, for example, a dispersion liquid, can be provided.

### MODES FOR CARRYING OUT THE INVENTION

### Composition for Oral Administration

The composition for oral administration according to the invention is a composition for oral administration that includes a water-insolubilized material including a bitter or astringent substance and a trivalent iron salt as a polyvalent metal salt, and a dispersant.

In the composition for oral administration according to the invention, bitterness or astringency due to a bitter or astringent substance is reduced (masked) by formation of a water-insoluble solid, i.e., a water-insolubilized material, from the bitter or astringent substance caused by the a polyvalent metal salt. In a case in which the dispersion medium is an aqueous medium, since the composition for oral administration includes the water-insolubilized material and a dispersant, the water-insolubilized material forms some of the dispersed particles, and a dispersion liquid that exhibits transparency not impairing the outer appearance can be obtained.

"A bitter or astringent substance" as used in the invention generically means a substance capable of exhibiting bitterness or astringency in a gustatory test on a human subject. Therefore, in the invention, the term "bitter substance" and the term "astringent substance" can be used interchangeably, and when these terms are used, these terms do not need to be recognized as clearly different concepts.

The scope of the term "a process" as used herein includes not only a discrete process, but also includes a process which cannot be clearly distinguished from another process, as long as an expected effect of the process of interest can be achieved.

Further, numerical ranges expressed by "(from) ... to ..." mentioned herein indicates a range including the numerical values described before and after "to" as a minimum value and a maximum value, respectively.

In a case in which the amount of a component in the composition is indicated in the invention, when there are plural substances corresponding to the component in the composition, the indicated amount means the total amount of the plural substances present in the composition, unless specifically stated otherwise.

The invention is described below.

### Water-Insolubilized Material

The composition for oral administration according to the invention includes a water-insolubilized material including a bitter or astringent substance and a polyvalent metal salt. Since the bitter or astringent substance included in the composition for oral administration is in the form of a water-insolubilized material, bitterness or astringency due to the bitter or astringent substance is effectively reduced (masked).

The "water-insolubilized material" in the invention means a fine solid that does not completely dissolve in water at 25°C. The presence of the water-insolubilized material can be confirmed using the particle size measurement apparatus described below. The presence of the water-insolubilized material can alternatively be determined by separating the fine solid or particles through ultrafiltration or dialysis.

The water-insolubilized material included in the composition for oral administration is in a form determined in accordance with the form of the composition for oral administration.

For example, in a case in which the composition for oral administration is in the form of a dispersion liquid, the water-insolubilized material is included in dispersed particles. In this case, there is no limitation as long as the water-insolubilized material forms at least a part of the dispersed particles. That is, the water-insolubilized material may be included inside the dispersed particles, or may be contained in such a manner that a part of the water-insolubilized material is exposed on the surface of the dispersed particles. Since the composition for oral administration is in the form of a dispersion liquid, and the water-insolubilized material is included in dispersed particles, transparency that does not impair the outer appearance can be exhibited.

Further, in a case in which the composition for oral administration is in powder form or in solid form, the water-insolubilized material forms a part of the powder or a part of the solid. Similar to the dispersion liquid described above, the water-insolubilized material in the composition for oral administration in powder form or in solid form will be included in dispersed particles dispersed in an aqueous medium in the case of mixing the composition for oral administration in powder form or in solid form with an aqueous medium. The composition for oral administration in the form of a dispersion liquid after combined with the aqueous medium can exhibit transparency that does not impair the outer appearance.

The size of the water-insolubilized material varies depending on the form of the composition for oral administration. In a case in which the composition for oral administration is in the form of a dispersion liquid, the size of the water-insolubilized material is the same as that of the dispersed particles described below. In a case in which the composition for oral administration is in powder form or in solid form, the size of the powder or the solid is not particularly limited, and may be a size with which dispersed particles having the size described below can be provided when the composition for oral administration in powder form or in solid form is contacted with an aqueous medium to form a dispersion liquid.

### Bitter or Astringent Substance

A bitter or astringent substance included in the water-insolubilized material may be any substance that can form, together with the polyvalent metal salt described below, a substance that does not dissolve in water at 25°C.

Examples of the bitter substance in the invention include acetaminophen, ampicillin, azithromycin, phenylthiourea, quinine, strychnine, nicotine, caffeine, naringin, limonin, ipomeamarone, cucurbitacin, iso-α acids (such as isohumulone, isocohumulone, and isoadhumulone), β-acids (such as lupulone, colprone, and adlupulone), catechin, bitter peptides (such as Gly-Leu and Leu-Phe), tannic acid, coffeic acid, gallic acid, and quercetin. Examples of the astringent substance include catechin (such as epigallocatechin gallate and epicatechin gallate), α-acids (such as humulone, cohumulone, and adhumulone), and chlorogenic acid. These bitter or astringent substances may be used singly, or in combination of two or more thereof. Further, these bitter or astringent substances may be used in the form of purified products, or in the form of, for example, extracts from natural ingredients.

The bitter or astringent substance preferably includes an acidic bitter or astringent substance, more specifically an organic acid. A high masking effect can be obtained when a bitter or astringent substance that is an organic acid is used.

Here, examples of the organic acid include carboxylic acid compounds, phenol compounds, enol compounds, and thiol compounds. Among the substances described above, specifically, α-acids, iso-α acids, β-acids, catechin, epigallocatechin gallate, epicatechin gallate, tannic acid, coffeic acid, gallic acid, quercetin, and chlorogenic acid are organic acids. The bitter or astringent substance in the invention is preferably at least one selected from the group consisting of carboxylic acid compounds such as tannic acid, coffeic acid, gallic acid, and chlorogenic acid, and enol compounds such as α-acids, iso-α acids, and β-acids. In particular, iso-α acids can suitably be used. Iso-α acids may be used in any form, and are easily available as hop extracts containing iso-α acids.

A single organic acid selected from those described above, or a combination of two or more organic acids selected from those described above, may be used as the bitter or astringent substance. For example, the bitter or astringent substance may include a combination of an iso-α acid and an α-acid. In the composition for oral administration that includes a combination of an iso-α acid and an α-acid, bitterness or astringency is reduced, the outer appearance in the case of being formed into a dispersion liquid is not impaired, and the stability of the iso-α acid against heating and storage stability of the iso-α acid can be made excellent.

In a case in which an iso-α acid and an α-acid are combined with each other, there is no particular limitation on the quantitative ratio therebetween. For example, the mass of α-acid to be combined may be from 0.5 times to 10 times the mass of iso-α acid.

The content of the bitter or astringent substance in the composition for oral administration may be varied, as appropriate, depending on the type or function of the bitter or astringent substance, or depending on the form of the composition for oral administration. For example, in a case in which the composition for oral administration is in the form of a dispersion liquid, the content of the bitter or astringent substance may generally be set to be from 0.001% by mass to 1% by mass of the total mass of the composition for oral administration.

### Polyvalent Metal Salt

The polyvalent metal salt included in the water-insolubilized material includes at least a trivalent iron salt as a divalent or higher-valent metal salt; monovalent metal salts do not exert a sufficient effect in terms of masking bitter or astringent substances. The polyvalent metal salt acts as a component that forms the water-insolubilized material. In a case in which the composition for oral administration is in the form of a dispersion liquid, a portion of the polyvalent metal salt that does not form dispersed particles including the water-insolubilized material may be present in the system.

The polyvalent metal salt may be a polyvalent metal salt of an inorganic compound or a polyvalent metal salt of an organic compound such as a carboxylic acid. Inorganic polyvalent metal salts and organic polyvalent metal salts are both capable of providing an expected masking effect.

Examples of the iron salt include ferric ammonium citrate, ferric pyrophosphate and ferric chloride. These may be used singly, or in combination of two or more thereof.

From the viewpoint of a masking effect, the content of polyvalent metal salt in the composition for oral administration is preferably from 0.01 times to 100 times, more preferably from 0.1 times to 50 times, and particularly preferably from 0.2 times to 10 times the content of bitter or astringent substance in terms of a mass ratio. When the content (mass) of polyvalent metal salt is equal to or greater than 0.01 times the content (mass) of bitter or astringent substance, a sufficient masking effect can be obtained. When the content (mass) of polyvalent metal salt is equal to or smaller than 100 times the content (mass) of bitter or astringent substance, an influence from an unpleasant taste of the polyvalent metal salt itself can be suppressed.

In addition to the range of the content (mass) of polyvalent metal salt specified relative to the content (mass) of bitter or astringent substance, the content of polyvalent metal salt in the composition for oral administration is preferably from 0.01 times to 100 times, more preferably from 0.05 times to 50 times, more preferably from 0.1 times to 10 times, and particularly preferably 0.2 times to 3 times the content (mol) of bitter or astringent substance in terms of a molar ratio, from the viewpoint of more favorable masking effect based on the reactivity between the polyvalent metal salt and the bitter or astringent substance. When the content (mol) of polyvalent metal salt is equal to or greater than 0.01 times the content (mol) of bitter or astringent substance, a sufficient masking effect can be obtained. When the content (mol) of polyvalent metal salt is equal to or smaller than 100 times the content (mol) of bitter or astringent substance, an influence from an unpleasant taste of the polyvalent metal salt itself can be suppressed.

### Dispersant

The dispersant in the invention is used in order to form dispersed particles that includes the water-insolubilized material including the bitter or astringent substance and the polyvalent metal salt when an aqueous medium is used as the dispersion medium, and in order to improve the dispersion stability of the formed dispersed particles in the system. The absorptivity of the bitter or astringent substance into the body is also improved by including the bitter or astringent substance in the dispersed particles.

A substance capable of dispersing the insolubilized material as a part of dispersed particles in an aqueous medium corresponds to the dispersant. Examples of the dispersant include water-soluble macromolecules, surfactants, clay substances, latexes, and clathrate compounds, which are preferable from the viewpoint of the dispersion stability of the dispersed particles including the water-insolubilized material. The dispersant is particularly preferably at least one selected from the group consisting of water-soluble macromolecules and surfactants. These dispersants may be used singly, or in combination of two or more thereof.

Examples of water-soluble macromolecules include proteins, polysaccharides, and synthetic macromolecules. There is no particular limitation on the molecular weight of the water-soluble macromolecule. In general, the molecular weight of the water-soluble macromolecule may be set to be in a range of from 1,000 to 1,000,000, and more preferably in a range of from 3,000 to 300,000, from the viewpoint of dispersion stability.

Examples of proteins include gelatin, albumin, casein, and zein. Examples of polysaccharides include cellulose, amylose, amylopectin, dextrin, dextran, pullulan, inulin, galactan, mannan, xylan, arabinan, glucomannan, galactomannan, pectic acid, alginic acid, agarose, agar, carrageenan, fucoidan, hyaluronic acid, chondroitin sulfate, heparin, gellan gum, native gelan gum, xanthan gum, chitin, chitosan, and derivatives thereof, for example, cellulose derivatives (such as hydroxypropylcellulose, hydroxypropyl methylcellulose, methylcellulose, ethyl cellulose, and carboxymethylcellulose), modified starches (such as hydroxypropyl starch, starch octenylsuccinate, and carboxymethyl starch), and alginic acid derivatives (such as propylene glycol alginate). Examples further include gum arabic, karaya gum, and ghatti gum. Examples of synthetic macromolecules include polyvinyl pyrrolidone and polyvinyl alcohol. These may be used singly, or in combination of two or more thereof.

Among these water-soluble macromolecules, gelatin, cellulose derivatives, modified starches, gum arabic, and pullulan are particularly preferable from the viewpoint of dispersion stability in the case of forming the composition for oral administration into a dispersion.

The surfactant may be any surfactant usable for oral administration, and examples thereof include surfactants generally known as emulsifying agents. Examples of the surfactants include fatty acid glycerides (for example, oleic acid monoglyceride, caprylic acid monoglyceride, lauric acid monoglyceride, capric acid monoglyceride, and caprylic acid diglyceride), polyglyceryl fatty acid esters (for example, diglyceryl monostearate, decaglyceryl decaoleate, pentaglyceryl trioleate, decaglyceryl monolaurate, decaglyceryl monomyristate, decaglyceryl monooleate, decaglyceryl monostearate, decaglyceryl distearate, pentaglyceryl monolaurate, pentaglyceryl monooleate, and pentaglyceryl monostearate), polyglyceryl polyricinoleates (for example, condensed tetraglyceryl ricinoleate and condensed hexaglyceryl ricinoleate), organic acid monoglycerides (for example, glyceryl monostearate lactate, glyceryl monostearate citrate, glyceryl monooleate citrate, and glyceryl monostearate succinate), sorbitan fatty acid esters (for example, sorbitan monolaurate, sorbitan monopalmitate, sorbitan monostearate, sorbitan tristearate, sorbitan monooleate, and sorbitan trioleate), propylene glycol fatty acid esters (for example, propylene glycol monostearate and propylene glycol monooleate), polysorbates (for example, polyoxyethylene sorbitan monolaurate, polyoxyethylene sorbitan monooleate, and polyoxyethylene sorbitan monostearate), sucrose fatty acid esters (for example, sucrose dioleic acid ester, sucrose distearic acid ester, sucrose dipalmitic acid ester, sucrose dimyristic acid ester, sucrose dilauric acid ester, sucrose monooleic acid ester, sucrose monostearic acid ester, sucrose monopalmitic acid ester, sucrose monomyristic acid ester, and sucrose monolauric acid ester), poloxamers (polyoxyethylene-polyoxypropylene copolymer), polyoxyethylene fatty acid esters (for example, polyoxyethylene castor oil, polyoxyethylene hydrogenated castor oil, and polyoxyethylene-12-hydroxystearic acid), phospholipids (for example, lecithin, lysolecithin, lysophosphatidic acid, and enzymatically treated lecithin), sodium alkylsulfates (for example, sodium lauryl sulfate), cholic acids (for example, sodium cholate, sodium deoxycholate, and sodium ursodeoxycholate), and saponin. These may be used singly, or in combination of two or more thereof. Lecithin, lysolecithin, and lysophosphatidic acid are particularly preferable surfactants from the viewpoint of an effect in terms of suppressing bitterness or astringency.

Examples of a clay substance that can be used as the dispersant include kaolin, talc, smectite, and hydrotalcite. Examples of a clathrate compound which can be used as the dispersant include cyclodextrin, cycloamylose, cluster dextrin, and derivatives thereof. These may be used singly, or in combination of two or more thereof.

From the viewpoint of the transparency and the stability of dispersed particles when the composition for oral administration is formed into a dispersion liquid, the dispersant is preferably a water-soluble macromolecule, and more preferably gelatin, gum arabic, a modified starch, a cellulose derivative, pullulan, or any combination thereof.

From the viewpoint of the dispersion stability when the composition for oral administration is formed into a liquid, the content of dispersant in the composition for oral administration is preferably from 0.1 times to 100 times, more preferably from 0.5 times to 50 times, and particularly preferably from 1 times to 25 times the content of bitter substance or astringent substance in terms of a mass ratio. When the content of dispersant is equal to or greater than 0.1 times the content of bitter substance or astringent substance in terms of a mass ratio, sufficient dispersion stability can be obtained, and generation of precipitates can be prevented when the composition is used in the form of a water dispersion. When the content of dispersant is equal to or smaller than 100 times the mass of bitter or astringent substance in terms of a mass ratio, an influence from an unpleasant taste of the dispersant itself can be suppressed.

The composition for oral administration according to the invention preferably has a configuration selected from the following configurations, from the viewpoint of masking a bitter or astringent substance or the stability in the case of forming the composition into a dispersion liquid.
(1) A composition for oral administration including: at least one bitter or astringent substance selected from the group consisting of an α-acid, an iso-α acid, a β-acid, catechin, epigallocatechin gallate, epicatechin gallate, tannic acid, coffeic acid, gallic acid, quercetin, and chlorogenic acid; at least one trivalent iron salt; and a dispersant.
(2) A composition for oral administration including: at least one bitter or astringent substance selected from the group consisting of an α-acid, an iso-α acid, a β-acid, catechin, epigallocatechin gallate, epicatechin gallate, tannic acid, coffeic acid, gallic acid, quercetin, and chlorogenic acid; at least one trivalent iron salt, the content of the iron salt being from 0.1 to 50 times the content of the at least one bitter or astringent substance in terms of a mass ratio, and being from 0.1 times to 10 times the content of the at least one bitter or astringent substance in terms of a molar ratio; and a dispersant, the content of dispersant being from 1 times to 25 times the content of the at least one bitter or astringent substance in terms of a mass ratio.
(3) A composition for oral administration including: a bitter or astringent substance that is a hop extract; at least one trivalent iron salt, the content of the at least one trivalent iron salt being from 0.1 to 50 times the content of the bitter or astringent substance in terms of a mass ratio and being from 0.1 times to 10 times the content of the bitter or astringent substance in terms of a molar ratio; and a dispersant, the content of dispersant being from 1 times to 25 times the content of the bitter or astringent substance in terms of a mass ratio;
(4) A composition for oral administration including: a bitter or astringent substance that is a hop extract; at least one trivalent metal salt selected from the group consisting of ferric ammonium citrate, ferric chloride and ferric pyrophosphate; and a dispersant;
(5) A composition for oral administration including: at least one bitter or astringent substance selected from the group consisting of an α-acid, an iso-α acid, a β-acid, catechin, epigallocatechin gallate, epicatechin gallate, tannic acid, coffeic acid, gallic acid, quercetin, and chlorogenic acid; at least one trivalent iron salt, the content of the at least one trivalent metal salt being from 0.1 to 50 times the content of the at least one bitter or astringent substance in terms of a mass ratio and being from 0.1 times to 10 times the content of the at least one bitter or astringent substance in terms of a molar ratio; and a dispersant;
(6) A composition for oral administration including: at least one bitter or astringent substance selected from the group consisting of an iso-α acid, a β-acid, catechin, epigallocatechin gallate, epicatechin gallate, tannic acid, coffeic acid, gallic acid, quercetin, and chlorogenic acid; at least one trivalent metal salt selected from the group consisting of ferric ammonium citrate, ferric chloride and ferric pyrophosphate, the content of the at least one trivalent metal salt being from 0.1 to 50 times the content of the at least one bitter or astringent substance in terms of a mass ratio and being from 0.1 times to 10 times the content of the at least one bitter or astringent substance in terms of a molar ratio; and at least one dispersant selected from the group consisting of gelatin, cellulose derivatives, modified starch, gum arabic, and pullulan, the content of the at least one dispersant being from 1 times to 25 times the content of the at least one bitter or astringent substance in terms of a mass ratio.

The metal salt in each of the compositions (1) to (6) for oral administration described above may include ferric chloride, from the viewpoint of stability and favorable outer appearance when the composition is formed into a dispersion.

The average particle diameter of the dispersed particles in the dispersion configuration of any of the compositions (1) to (6) for oral administration may be set to be from 1 nm to 500 nm, or from 1 nm to 300 nm, or from 1 nm to 150 nm, from the viewpoint of stability and favorable outer appearance when the composition is formed into a dispersion. Further, in this configuration, the metal salt may be specified to be at least one of ferric chloride or potassium alum.

The bitter or astringent substance in each of the compositions (1) to (6) for oral administration may include a combination of an iso-α acid and an α-acid. In a case in which the composition for oral administration contains a combination of an iso-α acid and an α-acid, from the viewpoint of stability and favorable outer appearance when the composition is formed into a dispersion, the metal salt may be configured such that the metal salt includes at least one of ferric chloride or potassium alum and such that the average particle diameter of the dispersed particles in the dispersion is from 1 nm to 500 nm, or from 1 nm to 300 nm, or from 1 nm to 150 nm.

### Other Components

In addition to the components described above, the composition for oral administration according to the invention may contain other components that are usually used in compositions for oral administration, in usually-employed amounts. Examples of the *other* components include other functional components, sweeteners, flavors, and pH adjustors.

A *functional component* means a component which is expected to exert a desired physiological effect in a living organism when the component is present in the living organism. Examples of the functional component include: minerals; oil-soluble or water-soluble vitamins such as vitamin E; functional amino sugars such as N-acetyl glucosamine; and functional mucopolysaccharides such as hyaluronic acid and chondroitin sulfate.

The sweetener may be any material that provides a sweet taste. Examples thereof include fructose, saccharides, and artificial sweeteners.

Examples of saccharides include: monosaccharides such as glucose, fructose, galactose, and high-fructose corn syrup; disaccharides such as sucrose, lactose, and paratinose; oligosaccharides such as fructo-oligosaccharide, isomalto-oligosaccharide, galacto-oligosaccharide, and paratinose; monosaccharide alcohols such as erythritol, sorbitol, xylitol, and mannitol; disaccharide alcohols such as maltitol, isomaltitol, and lactitol; trisaccharide alcohols such as maltotriitol, isomaltotriitol, and panitol; tetra- or higher-saccharide alcohols such as oligosaccharide alcohols; and sugar alcohols such as powdery starch syrup of maltitol.

Examples of the artificial sweetener include stevia, aspartame, saccharin, glycyrrhizin, thaumatin, and sucralose.

Examples of the flavor include natural flavors and synthetic flavors. Examples of natural flavors include flavor component-containing materials prepared according to ordinary methods using, for example, a grass root, bark, a flower, a fruit, pericarp, or another animal or plant as an ingredient. The scope of natural flavors also includes essential oils separated from natural materials by processing using a steam distillation method, a squeezing method, or an extraction method.

There is no particular limitation on the pH adjustor. Examples of preferable pH adjustors include citric acid, trisodium citrate, gluconic acid, L-tartaric acid, malic acid, lactic acid, adipic acid, succinic acid, acetic acid, and derivatives thereof. These may be used singly, or in combination of two or more thereof. However, the scope of the pH adjuster does not include ascorbic acid, ascorbic acid salts, and derivatives thereof. The pH adjustor is more preferably citric acid, gluconic acid, malic acid, lactic acid, or a derivative thereof.

In a case in which the composition for oral administration is formed into a powdery form, the composition may include a known excipient in order to obtain suitability for tableting or suitability for micro-granulation.

The composition for oral administration may include one excipient singly, or include two or more excipients in combination. The excipient may be any water-soluble substance that is generally used as an excipient, and examples thereof include: monosaccharides and polysaccharides such as glucose, fructose, lactose, maltose, sucrose, dextrin, maltodextrin, cyclodextrin, maltose, trehalose, and polysaccharide thickeners such as gum arabic, guar gum, pectin, and pullulan; sugar alcohols such as sorbitol, mannitol, maltitol, lactose, maltotriitol, and xylitol; inorganic salts such as sodium chloride and sodium sulfate; cellulose derivatives such as hydroxyethyl cellulose and hydroxypropylcellulose; starch derivatives obtained by subjecting starch to esterification, etherification, or termini reduction treatment; and modified starches, gelatin decomposition products, agar, and polyvinyl alcohol. Among these, monosaccharides, polysaccharides, sugar alcohols, and inorganic salts are preferable from the viewpoint of solubility.

Additives generally used for foods and drinks may also be added, such as lubricants, coloring agents, preservatives, thickening and stabilizing agents, antioxidants, color-producing agents, bleaching agents, antifungal agents, enzymes, brighteners, acidulants, seasoning agents, reinforcing agents, food manufacturing agents, and spice extracts.

These components may be used singly, or in combination of two or more thereof.

The state of the composition for oral administration may be any of liquid, solid, or powder.

The composition for oral administration in liquid form is a liquid that includes an aqueous medium such as water and dispersed particles (a dispersion liquid), and the dispersed particles include the water-insolubilized material.

In general, a particle size of more than 500 nm increases scattering light and decreases transparency; therefore, the average particle diameter of the dispersed particles including the water-insolubilized material is preferably 500 nm or less, more preferably 400 nm or less, still more preferably 300 nm or less, even more preferably 150 nm or less, and particularly preferably 100 nm or less, from the viewpoint of obtaining transparency that does not impair the outer appearance when used in drinks or the like. The average particle diameter of the dispersed particles is generally 1 nm or more. A particle diameter of from 1 nm to 500 nm improves the transparency of a liquid containing the dispersed particles, and also reduces sedimentation over time.

The average particle diameter of the dispersed particles can be measured with a commercially available particle size distribution measurement instrument or the like. Known methods for measuring a particle size distribution include optical microscopy, confocal laser microscopy, electron microscopy, atomic force microscopy, a static light scattering method, a laser diffraction method, a dynamic light scattering method, a centrifugal sedimentation method, an electrical pulse measurement method, a chromatographic method, and an ultrasonic attenuation method. Instruments corresponding to the respective principles are commercially available.

A dynamic light scattering method is applied to particle diameter measurement in the invention, due to its particle diameter range and ease of measurement. Examples of commercially available measurement instruments using a dynamic light scattering method include a NANOTRAC UPA (Nikkiso Co., Ltd.), a dynamic light scattering particle size distribution measurement instrument LB-550 (Horiba, Ltd.), and a fiber-optics particle size analyzer FPAR-1000 (Otsuka Electronics Co., Ltd.).

The average particle diameter of the dispersed materials in the invention is a value measured using a FPAR-1000. Specifically a median diameter of a scattering intensity distribution obtained by the Contin method is used as the particle diameter.

The composition for oral administration in powder or solid form is obtained by drying the composition for oral administration in liquid form, and is essentially composed of the components, other than an aqueous medium, contained in the composition for oral administration in liquid form. The composition for oral administration in solid form is formed by, for example, further binding the particles of the composition for oral administration in powder form, for example, by tableting.

### pH

There is no particular limitation on the pH of the composition for oral administration. From the viewpoints of an effect in terms of suppressing bitterness or astringency, the ease of ingestion, and storage stability, the pH of the composition for oral administration is preferably acidic; in particular, the pH is preferably from 2.0 to 6.0 at 20°C, and more preferably from 3.0 to 5.0 at 20°C.

### Method for Producing Composition for Oral Administration

A method for producing the composition for oral administration according to the invention includes combining a bitter or astringent substance, a trivalent iron salt, and a dispersant with an aqueous medium such as water. The production method is not particularly limited as long as the method includes a process of combining the components described above to obtain a composition for oral administration in liquid form.

The process of combining may include an emulsification or dispersion process of emulsifying or dispersing the respective components. The emulsification or dispersing of the respective components may be carried out using an ordinary emulsification apparatus which utilizes shearing actions, such as a stirrer, an impeller stirrer, a homomixer, or a continuous flow shearing apparatus, or a high pressure homogenizer, or an ultrasonic dispersion device. In particular, in order to produce fine particles, it is preferable to use a high pressure homogenizer or an ultrasonic dispersion device. The temperature at the time of the mixing is not particularly limited, and is preferably from 10°C to 100°C from the viewpoint of the stability of active ingredients.

Another method for producing the composition for oral administration according to the invention that may be employed is a precipitation method using a neutralization reaction. Specifically, this method includes mixing an alkaline solution of a bitter or astringent substance with an acidic solution of a trivalent iron salt. Particles (water-insolubilized material) can be obtained by carrying out the mixing of the alkaline solution and the acidic solution, for example, rapidly. In this method, a dispersant may be added to one of the solutions, or may be added after particles are formed by mixing the alkaline solution and the acidic solution.

To the mixing of the solution of a bitter or astringent substance and the solution of a trivalent iron salt, conditions usually applied to mixing of solutions may be applied. For example, one of the solutions may be added, while agitating, to the other solution in a temperature condition of from 4°C to 50°C, preferably from 4°C to 30°C. In order to control the particle diameter to be within a desired range, it is preferable to carry out rapid and uniform agitation, and it is preferable to add, to one solution that is being agitated, the other solution at a constant rate.

In a case in which the composition for oral administration in powder or solid form is to be produced, a composition in powder form can be obtained by adding a process of drying the composition for oral administration in liquid form by, for example, spray drying.

The drying means may be a known drying means, and examples thereof include air drying, heat drying, heated air drying, high frequency drying, ultrasonic drying, reduced pressure drying, vacuum drying, freeze drying, and spray drying. These means may be used singly, or in combination of two or more thereof.

The composition for oral administration may be shaped into a predetermined shape. The shaping of the composition for oral administration may be carried out by, for example, tableting the composition for oral administration in powder form. The tableting method is not particularly limited, and methods commonly used for this purpose may be used as they are. The shape into which the composition for oral administration is to be shaped is not particularly limited.

### Applications

The composition for oral administration can be used in various applications in accordance with the form thereof. For example, in a case in which the composition for oral administration is formed into a liquid form, i.e., a dispersion liquid containing dispersed particles including the water-insolubilized material, the composition for oral administration can favorably be used as drinks or the like having high transparency. In this case, a bottled drink or a liquid medicine can be prepared by filling a container with the composition for oral administration before or after the composition is subjected to heat sterilization or the like.

In a case in which the composition for oral administration is formed into a powder or solid form, the composition for oral administration can be applied, in the power or solid form or after dispersed in another aqueous medium when required, to applications involving oral ingestion. The composition for oral administration may also be used as a tablet or a capsule, in which case the composition for oral administration is expected to easily micro-disperse within the digestive tract.

In this case, the composition for oral administration in powder or solid form may be contained in separate containers or compartments, whereby powdered or solid food or drink or medicine can be obtained. In a case in which the composition for oral administration is prepared as food or medicine to be ingested in powder or solid form, the food or medicine is easy to ingest because the bitterness or astringency of the bitter or astringent substance is effectively masked even when the food or medicine is collapsed in the mouth.

### Method for Suppressing Bitterness or Astringency

The method for suppressing bitterness or astringency according to the invention is a method for suppressing bitterness or astringency of a composition containing a bitter or astringent substance, and the method includes combining the bitter or astringent substance with a trivalent iron salt and a dispersant.

According to this method for suppressing bitterness or astringency, the bitterness or astringency of the bitter or astringent substance is reduced (masked) because the bitter or astringent substance is insolubilized by the trivalent iron salt to form a water-insolubilized material, which constitutes a part of dispersed particles in the liquid due to the inclusion of the dispersant.

With regard to the bitter or astringent substance, the trivalent iron salt, and the dispersant used in the method for suppressing bitterness or astringency, the explanations described above are directly applicable, and preferable embodiments of each component are also the same as those described above.

For example, the method for suppressing bitterness or astringency according to the invention is preferably any of the following embodiments, from the viewpoint of improved masking effect:
(1) A method including combining at least one bitter or astringent substance selected from the group consisting of an α-acid, an iso-α acid, a β-acid, catechin, epigallocatechin gallate,
   epicatechin gallate, tannic acid, coffeic acid, gallic acid, quercetin, and chlorogenic acid, with a dispersant and a trivalent iron salt.
(2) A method including combining at least one bitter or astringent substance selected from the group consisting of an α-acid, an iso-α acid, a β-acid, catechin, epigallocatechin gallate,
   epicatechin gallate, tannic acid, coffeic acid, gallic acid, quercetin, and chlorogenic acid, with a dispersant and a trivalent iron salt,
   the content of the trivalent iron salt being from 0.1 to 50 times the content of the at least one bitter or astringent substance in terms of a mass ratio and being from 0.1 times to 10 times the content of the at least one bitter or astringent substance in terms of a molar ratio, and
   the content of dispersant being from 1 times to 25 times the content of the at least one bitter or astringent substance in terms of a mass ratio.
(3) A method including combining a bitter or astringent substance that is a hop extract, with a dispersant and at least one trivalent iron salt,
   the content of the at least one trivalent iron salt being from 0.1 to 50 times the content of the bitter or astringent substance in terms of a mass ratio and being from 0.1 times to 10 times the content of the bitter or astringent substance in terms of a molar ratio,
   the content of dispersant being from 1 times to 25 times the content of the bitter or astringent substance in terms of a mass ratio.
(4) A method including: combining a bitter or astringent substance that is a hop extract, with a dispersant and at least one trivalent iron salt selected from the group consisting of ferric ammonium citrate, ferric chloride and ferric pyrophosphate.
(5) A method including: combining at least one bitter or astringent substance selected from the group consisting of an α-acid, an iso-α acid, a β-acid, catechin, epigallocatechin gallate, epicatechin gallate, tannic acid, coffeic acid, gallic acid, quercetin, and chlorogenic acid, with a dispersant and at least one trivalent iron salt,
   the content of the at least one trivalent metal salt being from 0.1 to 50 times the content of the at least one bitter or astringent substance in terms of a mass ratio and being from 0.1 times to 10 times the content of the at least one bitter or astringent substance in terms of a molar ratio.
(6) A method including combining at least one bitter or astringent substance selected from the group consisting of an iso-α acid, a β-acid, catechin, epigallocatechin gallate, epicatechin gallate, tannic acid, coffeic acid, gallic acid, quercetin, and chlorogenic acid, with at least one trivalent iron salt selected from the group consisting of ferric ammonium citrate, ferric chloride and ferric pyrophosphate, and at least one dispersant selected from the group consisting of gelatin, a cellulose derivative, a modified starch, gum arabic, and pullulan,
   the content of the at least one trivalent iron salt being from 0.1 to 50 times the content of the at least one bitter or astringent substance in terms of a mass ratio and from 0.1 times to 10 times the content of the at least one bitter or astringent substance in terms of a molar ratio,
   the content of the at least one dispersant being from 1 times to 25 times the content of the at least one bitter or astringent substance in terms of a mass ratio.

The metal salt in methods (1) to (6) for suppressing bitterness or astringency may include ferric chloride, from the viewpoints of stability and favorable outer appearance when formed into a dispersion.

In each of methods (1) to (6) for suppressing bitterness or astringency, the average particle diameter of the dispersed particles in the obtained composition for oral administration in the form of a dispersion may be set to be from 1 nm to 500 nm, or from 1 nm to 300 nm, or from 1 nm to 150 nm, from the viewpoint of stability and favorable outer appearance when the composition is formed into a dispersion. Further, in this configuration, the metal salt may be specified to be at least one of ferric chloride or potassium alum.

The bitter or astringent substance in each of methods (1) to (6) for suppressing bitterness or astringency may include a combination of an iso-α acid and an α-acid. In a case in which the bitter or astringent substance includes a combination of an iso-α acid and an α-acid, from the viewpoint of stability and favorable outer appearance of the obtained composition for oral administration in the form of a dispersion, the iron salt may be ferric chloride and such that the average particle diameter of the dispersed particles in the obtained composition for oral administration in the form of a dispersion is from 1 nm to 500 nm, or from 1 nm to 300 nm, or from 1 nm to 150 nm.

### EXAMPLES

The invention is described below in detail by way of examples. However, the invention is by no means limited thereto. Further, "% by mass" means "mass /mass%" and "% by volume" means "mass (g) / volume (mL) %", unless mentioned otherwise.

### Example 1

### Preparation of Dispersion Liquid 1

A 1% by mass aqueous solution of iso-α acid was obtained by diluting 1 g of a 30% by mass stock solution of iso-α acid (ISO-EXTRACT 30% manufactured by Hopsteiner) with 29g of purified water. Similarly, a 1% by mass aqueous solution of ferric chloride was obtained by diluting 1 g of ferric chloride hexahydrate (manufactured by Wako Pure Chemical Industries, Ltd., hereinafter abbreviated to ferric chloride) with 99 g of purified water.

Next, 6.42 g of purified water, 500 µL of the 1% by mass aqueous solution of ferric chloride, and 75.7 mg of gelatin (COLLAGEN PEPTIDE HBC-P20 having a molecular weight of from 15,000 to 40,000, manufactured by Nitta Gelatin Inc,) were added into a 10 mL vial in which a stir bar was placed, and the resultant mixture was agitated using a stirrer. 1 mL of the 1% by mass aqueous solution of iso-α acid was added thereto by being added into the water all at once. As a result, 8 mL of an iron dispersion liquid having an iso-α acid concentration of 0.13% by mass was obtained (dispersion liquid 1).

### Example 2

### Preparation of Dispersion Liquid 2

Dispersion liquid 2 was obtained in the same manner as that in Example 1, except that the gelatin used in Example 1 was replaced by gum arabic (INSTANTGUM AB having a molecular weight of 200,000, manufactured by Colloide Naturels International).

### Example 3

### Preparation of Dispersion Liquid 3

Dispersion liquid 3 was obtained in the same manner as that in Example 1, except that the gelatin used in Example 1 was replaced with pullulan (pullulan according to Japanese Pharmacopoeia and having a molecular weight of 70,000, manufactured by Hayashibara Biochemical Laboratories).

### Example 4

### Preparation of Dispersion Liquid 4

Dispersion liquid 4 was obtained in the same manner as that in Example 1, except that the gelatin used in Example 1 was replaced by hydroxypropylcellulose (manufactured by Tokyo Chemical Industry Co., Ltd., hereinafter abbreviated to HPC; molecular weight: 15,000 to 30,000).

### Example 5

### Preparation of Dispersion Liquid 5

Dispersion liquid 5 was obtained in the same manner as that in Example 1, except that the gelatin used in Example 1 was replaced by starch octenylsuccinate (EMULSTAR A1 having a molecular weight of 4500, manufactured by Matsutani Chemical Industry Co., Ltd.).

### Example 6

### Preparation of Dispersion Liquid 6

Dispersion liquid 6 was obtained in the same manner as that in Example 1, except that the gelatin used in Example 1 was replaced by polyvinyl alcohol (having a molecular weight of 66,000, manufactured by Wako Pure Chemical Industries, Ltd., ).

### Example 7

### Preparation of Dispersion Liquid 7

Dispersion liquid 7 was obtained in the same manner as that in Example 1, except that the gelatin used in Example 1 was replaced by lysolecithin (LPL-20S manufactured by Kewpie Corporation).

### Example 8 (Not in accordance with the invention)

### Preparation of Dispersion Liquid 8

Dispersion liquid 8 was obtained in the same manner as that in Example 1, except that the ferric chloride used in Example 1 was replaced by potassium alum dodecahydrate (manufactured by Wako Pure Chemical Industries, Ltd., hereinafter abbreviated to potassium alum).

### Example 9 (Not in accordance with the invention)

### Preparation of Dispersion Liquid 9

An aqueous solution containing 1% by mass of iso-α acid was obtained by adding a 0.1 N aqueous solution of sodium hydroxide to 0.04 g of a 30% by mass stock solution of iso-α acid (ISO-EXTRACT 30%, manufactured by Hopsteiner) and 0.048g of starch octenylsuccinate (EMULSTAR A1 manufactured by Matsutani Chemical Industry Co., Ltd.), thereby causing dissolution.

Next, 7 g of purified water, 1 ml of 0.1 N hydrochloric acid, and 1 ml of a 1% by mass aqueous solution of calcium chloride were added into a 10 mL vial in which a stir bar was placed, and the resultant mixture was agitated using a stirrer. 1 ml of the 1% by mass aqueous solution of iso-α acid was added thereto by being added into the water. As a result, a dispersion liquid having an iso-α acid concentration of 0.1% by mass was obtained (dispersion liquid 9).

### Example 10

### Preparation of Dispersion Liquid 10

A 5% by volume ethanol solution of iso-α acid was obtained by diluting 1 g of a 30% by mass stock solution of iso-α acid (ISO-EXTRACT 30%, manufactured by Hopsteiner,) and 566 mg of α-acid (YC-CO2 Hop Extract manufactured by YAKIMA Chief) with 4.5 mL of ethanol.

Similarly, a 5% by mass aqueous solution of ferric chloride was obtained by diluting 1 g of ferric chloride hexahydrate (manufactured by Wako Pure Chemical Industries, Ltd., hereinafter abbreviated to ferric chloride) with 19 g of purified water.

Next, 5.87 g of purified water, 750 µL of the 5% by mass aqueous solution of ferric chloride, and 378 mg starch octenylsuccinate (EMULSTAR A1 having a molecular weight of 4,500, manufactured by Matsutani Chemical Industry Co., Ltd.) were added into a 10 mL vial in which a stir bar was placed, and the resultant mixture was agitated using a stirrer. 1 mL of the 5% by volume ethanol solution of iso-α acid was added thereto all at once, as a result of which 8 mL of an iron dispersion liquid having an iso-α acid concentration of 0.63% by volume was obtained. Then, ethanol was distilled away therefrom, and 1 mL of purified water was further added thereto. As a result, a dispersion liquid having an iso-α acid concentration of 0.63% by mass was obtained (dispersion liquid 10).

### Example 11

### Preparation of Solid 1

1 gram of trehalose (manufactured by Hayashibara Co., Ltd.) was added and dissolved in 20 g of a dispersion liquid (dispersion liquid 5 obtained in Example 5). The resultant was dispensed into 5 mL brown vials in an amount of 1 mL each, and then the 5mL brown vials were cooled at -80°C for half a day. These were dried using a freeze dryer (AS ONE Corporation, VFD-03), as a result of which a powdered solid of an iron dispersion liquid was obtained (solid 1).

### Example 12

### Preparation of Solid 2

A powdered solid of an iron dispersion liquid (solid 2) was obtained in the same manner as that in Example 11, except that dispersion liquid 5 was replaced by dispersion liquid 10 obtained in Example 10.

### Comparative Example 1

### Preparation of Dispersion Liquid R1

Dispersion liquid R1 was obtained in the same manner as that in Example 1, except that the ferric chloride and the gelatin were not added in Comparative Example 1.

### Comparative Example 2

### Preparation of Dispersion liquid R2

Dispersion liquid R2 was obtained in the same manner as that in Example 1, except that the ferric chloride was not added in Comparative Example 2.

### Comparative Example 3

### Preparation of Dispersion Liquid R3

Dispersion liquid R3 was obtained in the same manner as that in Example 7, except that the ferric chloride was not added in Comparative Example 3.

### Comparative Example 4

### Preparation of Dispersion Liquid R4

Dispersion liquid R4 was obtained in the same manner as that in Example 1, except that the gelatin was not added in Comparative Example 4.

### Comparative Example 5

### Preparation of Dispersion Liquid R5

An aqueous solution containing 1% by volume of iso-α acid was obtained by diluting 0.04 g of a 30% by volume stock solution of iso-α acid (ISO-EXTRACT 30%, manufactured by Hopsteiner) and 0.096 g of y-cyclodextrin (manufactured by Wako Pure Chemical Industries, Ltd.) with a 0.1N aqueous solution of sodium hydroxide.

Next, 7.5 g of purified water and 1.5 ml of 0.1N hydrochloric acid were added into a 10 mL vial in which a stir bar was placed, and the resultant mixture was agitated using a stirrer. 1 ml of the 1% by volume aqueous solution of iso-α acid was added thereto by being added into the water, as a result of which a dispersion liquid having an iso-α acid concentration of 0.1% by mass (dispersion liquid R5) was obtained.

### Evaluation

Particle size measurement, evaluation of outer appearance, and evaluation of bitterness and astringency was carried out with respect to dispersion liquids 1 to 10 and R1 to R5 and Solids 1 and 2 obtained above. Evaluation of stability against heating and storage stability were also carried out with respect to dispersion liquids 5, 10 and R1 and Solids 1 and 2. The measurement methods were as follows.

Prior to carrying out the evaluation of bitterness and astringency, 45.0 mg of L-(+)-tartaric acid (manufactured by Wako Pure Chemical Industries, Ltd.) and 2.24 g of potassium chloride (manufactured by Wako Pure Chemical Industries, Ltd.) were dissolved in 900 mL of purified water, as a result of which a reference liquid (pH 3.5) was obtained.

8 mL each of dispersion liquids 1 to 8 and Dispersion liquids R1 to R4 obtained above were individually mixed with 92 g of the reference liquid, thereby providing sample solutions for evaluation. Further, 10 mL each of dispersion liquids 9 and R5 were individually mixed with 90 g of the reference liquid, thereby providing sample solutions for evaluation. The pH of each evaluation sample was pH 3.5.

2mL of dispersion liquid 10 was mixed with 98 g of the reference liquid, thereby providing a sample solution for evaluation. Further, 100 mg each of solids 1 and 2 were individually added to 1 mL of purified water and dissolved completely, and then 1g each of the resulting solutions were individually mixed with 49 g of the reference liquid, thereby providing sample solutions for evaluation.

### (1) Particle Size Measurement

Particle size measurement was carried out at 23°C using a dynamic light scattering particle size distribution measurement system FPAR-1000 (manufactured by Otsuka Electronics Co., Ltd.). The median diameter was used as the value of the average particle diameter. The results are shown in Table 1. Here, "nd" in Table 1 means that particles were not detected, indicating that the sample was in the dissolved state.

### (2) Evaluation of Outer Appearance

After the preparation of the sample solutions for evaluation, the sample solutions for evaluation were allowed to stand for 2 hours at 23°C, and the outer appearance of each sample solution was visually observed. Sample solutions in which precipitation was not observed after 2 hours was ranked A while sample solution in which precipitation was observed after 2 hours was ranked C. The results are shown in Table 1.

### (3) Evaluation of Stability over Time

After the preparation of the sample solutions for evaluation, the sample solutions for evaluation were allowed to stand at 50°C for 14 days, and then the outer appearance of each sample solution was visually observed. Sample solutions in which precipitation was not observed after 14 days was ranked A, and sample solutions in which precipitation was not observed after 7 days but observed after 14 days was ranked B, and sample solutions in which precipitation was observed after 7 days was ranked C. The results are shown in Table 1.

### (4) Evaluation of Bitterness and Astringency

Evaluation of bitterness and astringency was carried out using a taste sensing system SA402B (Intelligent Sensor Technology, Inc.).

Bitterness and astringency were obtained, through calculation, by conducting the following data analysis on the obtained measurement results. Specifically, errors among measurements were eliminated from the obtained measurement data by interpolated addition and interpolated correction using the reference liquid as a common sample. Further, differences in taste intensities sensed by humans were estimated based on the output of the sensor through estimate value calculation from the corrected data. The results are shown in Table 1.

### (5) Evaluation of Stability of Iso-α Acid against Heating

After the preparation of the sample solutions for evaluation, 4 mL each of the sample solutions for evaluation were individually dispensed into 5 mL brown vials, and heated in a thermostatic oven at 90°C for 10 minutes. Further, 100 mg each of solids 1 and 2 were individually added into 5mL brown vials, and heated in a thermostatic oven at 90°C for 10 minutes.

Each of dispersion liquid 5, 10 and R1 after heating, 0.1 N sodium hydroxide, and tetrahydrofuran were mixed in a volume ratio of 1:0.1:0.9, and agitated, thereby providing respective sample solutions for thermal evaluation.

1 mL of purified water was added to 100 mg each of solids 1 and 2, and each solid was completely dissolved. Further, the resultant solutions were individually mixed with 49 g of the reference liquid, thereby providing sample solutions. Each of the sample solutions, 0.1 1 N sodium hydroxide, and tetrahydrofuran were mixed in a volume ratio of 1:0.1:0.9, and agitated, thereby providing post-heating sample solutions.

Pre-heating sample solutions were also prepared in a similar manner, using dispersion liquids before the heating.

Each of the pre-heating sample solutions and the post-heating sample solutions was subjected to quantification of iso-α acid using a high performance liquid chromatograph (column: CAPCELLPAK C-18 manufactured by Shiseido Co., Ltd., detector: a UV detector, detection wavelength: 255 nm). A 30% by mass stock solution of iso-α acid (ISO-EXTRACT 30%, manufactured by Hopsteiner) was used as a reference sample. Stability against heating was evaluated by calculating the ratio between the iso-α acid areas of the pre-heating sample solution and the post-heated sample solution, and evaluated as a relative value assuming that the area ratio in the case of dispersion liquid 5 was 1. The results are shown in Table 2.

### (6) Evaluation of Storage Stability of Iso-α Acid

After the preparation of the sample solutions for evaluation, 4mL each of the sample solutions for evaluation were individually dispensed into 5 mL brown vials, and heated in a thermostatic oven at 90°C for 10 minutes. Further, 100 mg each of solids 1 and 2 were added into 5mL brown vials, and stored in a thermostatic oven at 45°C for 1 week.

Each of dispersion liquids 5, 10 and R1 after storage, 0.1 N sodium hydroxide, and tetrahydrofuran were mixed in a volume ratio of 1:0.1:0.9, and agitated, thereby providing respective sample solutions for thermal evaluation.

1 mL of purified water was added to 100 mg each of solids 1 and 2, and each solid was completely dissolved. Then, the resultant solutions were individually further mixed with 49 g of the reference liquid to give sample solutions. Each of the sample solutions, 0.1 N sodium hydroxide, and tetrahydrofuran were mixed in a volume ratio of 1:0.1:0.9, and agitated, thereby providing post-storage sample solutions.

Pre-storage sample solutions were also prepared in a similar manner, using dispersion liquids before the storage.

Each of the pre-storage samples solutions and post-storage sample solutions was subjected to quantification of iso-α acid using a high performance liquid chromatography (column: Shiseido Co., Ltd., CAPCELLPAK C-18, detector: a UV detector, detection wavelength: 255 nm). A 30% by mass stock solution of iso-α acid (ISO-EXTRACT 30%, manufactured by Hopsteiner) was used as a reference sample. Storage stability was evaluated by calculating the ratio between the iso-α acid areas of the pre-storage sample solution and the post-storage sample solution, and evaluated as a relative value assuming that the area ratio in the case of dispersion liquid 5 was 1. The results are shown in Table 2.

**Table 1**

| | Sample No. | Composition | | | [Metal]/[Bitter or astringent substance] Molar ratio | Particle diameter (nm) | Appearance | Stability over time | Bitterness evaluation score | Astringency evaluation score |
|---|---|---|---|---|---|---|---|---|---|---|
| | | Bitter or astringent substance | Metal salt | dispersant | | | | | | |
| Example 1 | Dispersion liquid 1 | iso-α acid | ferric chloride | gelatin | 0.67 | 79 | A | A | 1.8 | 0.9 |
| Example 2 | Dispersion liquid 2 | iso-α acid | ferric chloride | gum arabic | 0.67 | 215 | A | A | 1.8 | 0.9 |
| Example 3 | Dispersion liquid 3 | iso-α acid | ferric chloride | pullulan | 0.67 | 240 | A | A | 0.9 | 0.5 |
| Example 4 | Dispersion liquid 4 | iso-α acid | ferric chloride | HPC | 0.67 | 74 | A | A | 1.8 | 0.9 |
| Example 5 | Dispersion liquid 5 | iso-α acid | ferric chloride | starch octenylsuccinate | 0.67 | 95 | A | A | 1.4 | 0.5 |
| Example 6 | Dispersion liquid 6 | iso-α acid | ferric chloride | PVA | 0.67 | 127 | A | A | 1.6 | 0.8 |
| Example 7 | Dispersion liquid 7 | iso-α acid | ferric chloride | lysolecithin | 0.67 | 139 | A | B | 1.3 | 0.8 |
| * Example 8 | Dispersion liquid 8 | iso-α acid | potassium alum | gelatin | 0.67 | 57 | A | A | 6.6 | 5.1 |
| * Example 9 | Dispersion liquid 9 | iso-α acid | calcium chloride | starch octenylsuccinate | 0.67 | 111 | A | A | 8.4 | 7.3 |
| Example 10 | Dispersion liquid 10 | iso-α acid and α acid | ferric chloride | starch octenylsuccinate | 0.50 | 137 | A | A | 1.7 | 1.3 |
| Example 11 | Solid 1 | iso-α acid | ferric chloride | starch octenylsuccinate | 0.67 | 70 | A | A | 3.7 | 0.7 |
| Example 12 | Solid 2 | iso-α acid and α acid | ferric chloride | starch octenylsuccinate | 0.50 | 150 | A | A | 4.0 | 1.0 |
| Comparative Example 1 | Dispersion liquid R1 | iso-α acid | None | None | - | nd | A | A | 18.1 | 12.2 |
| Comparative Example 2 | Dispersion liquid R2 | iso-α acid | None | gelatin | - | nd | A | A | 12 | 10.8 |
| Comparative Example 3 | Dispersion liquid R3 | iso-α acid | None | lysolecithin | - | 375 | A | A | 14.4 | 5.3 |
| Comparative Example 4 | Dispersion liquid R4 | iso-α acid | ferric chloride | None | 0.67 | Precipitation | C | C | 0.4 | 0.2 |
| Comparative Example 5 | Dispersion liquid R5 | iso-α acid | None | γ-cyclodextrin | - | Precipitation | C | C | 8.5 | 6.8 |

| | | | | | | | | | | |
|---|---|---|---|---|---|---|---|---|---|---|
| * Reference Example | | | | | | | | | | |

As shown in Table 1, all of dispersion liquids 1 to 7 and 10, which contained both a ferric salt and a dispersant, did not exhibit precipitation, and bitterness evaluation scores thereof and astringency evaluation scores thereof were lower than those of dispersion liquid R1, which contained only iso-α acids. Particle diameter in dispersion liquid R1 could not be measured since dispersion liquid R1 contained neither a metal salt nor a dispersant, and dispersion liquid R1 exhibited a bitterness evaluation score of 18.1 and an astringency evaluation score of 12.2, which are very high scores. This indicates that iso-α acids completely dissolved and acted as a bitter and astringent component.

In particular, the results of the evaluation of dispersion liquid reveal that transparent outer appearance as well as better effects in terms of suppression of bitterness and astringency can be obtained by using ferric chloride, which is a trivalent metal salt.

Further, the results of the evaluation of dispersion liquid reveal that transparent outer appearance as well as suppression of bitterness and astringency can be achieved regardless of the type of the dispersant.

Dispersion liquid 10 including a combination of an iso-α acid and an α-acid also exhibited an effect in terms of suppression of bitterness and astringency.

In the case of solids 1 and 1 prepared by forming powders from dispersion liquids, precipitation was not observed even in a re-dispersion liquid in which each solid was redispersed in water, and the outer appearance of the re-dispersion liquid was transparent. It was also found that effects in terms of suppression of bitterness and astringency were maintained.

In contrast, it was demonstrated that in dispersion liquids R2, R3, and R5, to which a metal salt was not added, use of a dispersant alone cannot suppress bitterness and astringency.

Furthermore, in the case of dispersion liquid R4, to which a dispersant was not added, the dispersion liquid immediately after the preparation thereof was clouded, and the dispersion coagulated to generate precipitation before 2 hour passed. Since a supernatant of dispersion liquid R4 after the removal of precipitates exhibited a bitterness evaluation score of 0.4 and an astringency evaluation score of 0.2, it is understood that the metal salt effectively insolubilized iso-α acids, but the dispersion stability was not high. Therefore, drinks and foods obtained using dispersion liquid R4 would not have good outer appearance, and grainy sensation or the like would be felt in the mouth.

**Table 2**

| | Sample No. | Composition | | | [Metal]/[Bitter or astringent substance] Molar ratio | Evaluation score of iso-α-acid stability against heating | Evaluation score of iso-α-acid storage stability |
|---|---|---|---|---|---|---|---|
| | | Bitter or astringent substance | Metal Salt | Dispersant | | | |
| Example 5 | Dispersion liquid 5 | iso-α acid | ferric chloride | starch octenylsuccinate | 0.67 | 1.0 | 1.0 |
| Example 10 | Dispersion liquid 10 | iso-α acid and α acid | ferric chloride | starch octenylsuccinate | 0.50 | 2.1 | 4.8 |
| Example 11 | Solid 1 | iso-α acid | ferric chloride | starch octenylsuccinate | 0.67 | 2.1 | 4.0 |
| Example 12 | Solid 2 | iso-α acid and α acid | ferric chloride | starch octenylsuccinate | 0.50 | 2.1 | 4.8 |

As shown in Table 2, in addition to transparent outer appearance and production of excellent effects in terms of suppression of bitterness and astringency as described above, it was found that unexpected effects - enhanced stability of iso-α acid against heating and enhanced storage stability of iso-α acid - were exerted in a case in which an iso-α acid and an α-acid were used in combination as a bitter or astringent substance (dispersion liquid 10).

By comparison between dispersion liquid 5 and solid 1 prepared by forming powder from dispersion liquid 5, it was found that the conversion into the powdered state improves the stability of iso-α acid at the time of heating and at the time of storage, in addition to the transparent outer appearance and the production of an excellent effect in terms of suppression of bitterness and astringency as described above.

Examples 10 to 12 (dispersion liquid 10 and solids 1 and 2) exhibited 5% improvement of the stability against heating and from 5% to 26% improvement of the storage stability, as compared with Comparative Example 1 (dispersion liquid 1).

It is demonstrated that a composition for oral administration which has transparency that does not impair the outer appearance when formed into a dispersion liquid, and in which bitterness or astringency due to a bitter or astringent substance is reduced can be provided according to the invention.

## Claims

1. A composition for oral administration comprising:
a water-insolubilized material which does not completely dissolve in water at 25°C obtainable by combining a bitter or astringent substance and a trivalent iron salt; and
a dispersant.

2. A composition for oral administration according to Claim 1, wherein the bitter or astringent substance includes an organic acid.

3. A composition for oral administration according to Claim 1 or Claim 2, wherein the bitter or astringent substance includes a hop extract.

4. A composition for oral administration according to any preceding Claim, wherein the bitter or astringent substance includes an iso-α acid.

5. A composition for oral administration according to any preceding Claim, wherein the bitter or astringent substance includes a combination of an iso-α acid and an α-acid.

6. A composition for oral administration according to any preceding Claim, wherein the dispersant is a water-soluble macromolecule and/or a surfactant.

7. A composition for oral administration according to Claim 6, wherein the dispersant is a water-soluble macromolecule selected from gelatin, gum arabic, modified starch, pullulan and a cellulose derivative.

8. A composition for oral administration according to any preceding Claim, containing 0.01 to 100 moles of a polyvalent metal salt per mole of the bitter or astringent substance.

9. A composition for oral administration according to any preceding Claim, wherein the content of the dispersant is from 0.1 times to 100 times the content of the bitter or astringent substance in terms of a mass ratio.

10. A composition for oral administration according to any preceding Claim, which is in the form of a dispersion liquid including dispersed particles, and the dispersed particles include the water-insolubilized material.

11. A composition for oral administration according to Claim 10, wherein the average particle diameter of the dispersed particles is 300 nm or less.

12. A method for reducing bitterness or astringency of a composition that includes a bitter or astringent substance, the method including combining the bitter or astringent substance with a dispersant and a trivalent salt of iron.

## Patentansprüche

1. Zusammensetzung zur oralen Verabreichung, umfassend:
ein wasserunlösliches Material, das sich in Wasser bei 25°C nicht vollständig löst, erhältlich durch Kombinieren einer bitteren oder adstringierenden Substanz und eines trivalenten Eisensalzes; und
ein Dispergiermittel.

2. Zusammensetzung zur oralen Verabreichung gemäss Anspruch 1, wobei die bittere oder adstringierende Substanz eine organische Säure einschliesst.

3. Zusammensetzung zur oralen Verabreichung gemäss Anspruch 1 oder Anspruch 2, wobei die bittere oder adstringierende Substanz einen Hopfenextrakt einschliesst.

4. Zusammensetzung zur oralen Verabreichung gemäss irgendeinem der vorhergehenden Ansprüche, wobei die bittere oder adstringierende Substanz eine Iso-α-säure einschliesst.

5. Zusammensetzung zur oralen Verabreichung gemäss irgendeinem der vorhergehenden Ansprüche, wobei die bittere oder adstringierende Substanz eine Kombination aus einer Iso-α-säure und einer α-Säure einschliesst.

6. Zusammensetzung zur oralen Verabreichung gemäss irgendeinem der vorhergehenden Ansprüche, wobei das Dispergiermittel ein wasserlösliches Makromolekül und/oder ein Tensid ist.

7. Zusammensetzung zur oralen Verabreichung gemäss Anspruch 6, wobei das Dispergiermittel ei wasserlösliches Makromolekül, ausgewählt aus Gelatine, Gummi arabicum, modifizierter Stärke, Pullulan und einem Cellulosederivat, ist.

8. Zusammensetzung zur oralen Verabreichung gemäss irgendeinem der vorhergehenden Ansprüche, die 0,01 bis 100 mol eines polyvalenten Metallsalzes pro Mol der bitteren oder adstringierenden Substanz enthält.

9. Zusammensetzung zur oralen Verabreichung gemäss irgendeinem der vorhergehenden Ansprüche, wobei der Gehalt an Dispergiermittel das 0,1- bis 100-fache des Gehalts an bitterer oder adstringierender Substanz, bezogen auf das Masseverhältnis, beträgt.

10. Zusammensetzung zur oralen Verabreichung gemäss irgendeinem der vorhergehenden Ansprüche, die in Form einer Dispersionsflüssigkeit vorliegt, die dispergierte Partikel einschliesst, und die dispergierten Partikel schliessen das wasserunlösliche Material ein.

11. Zusammensetzung zur oralen Verabreichung gemäss Anspruch 10, wobei der durchschnittliche Partikeldurchmesser der dispergierten Partikel 300 nm oder weniger beträgt.

12. Verfahren zum Reduzieren der Bitterkeit und Adstringenz einer Zusammensetzung, die eine bittere oder adstringierende Substanz einschliesst, wobei das Verfahren das Kombinieren einer bitteren oder adstringierenden Substanz mit einem Dispergiermittel und einem trivalenten Eisensalz einschliesst.

## Revendications

1. Composition pour une administration orale comprenant :
un matériau insolubilisé dans l'eau qui ne se dissout pas complètement dans l'eau à 25°C pouvant être obtenu en combinant une substance amère ou astringente et un sel de fer trivalent ; et
un dispersant.

2. Composition pour une administration orale selon la revendication 1, dans laquelle la substance amère ou astringente inclut un acide organique.

3. Composition pour une administration orale selon la revendication 1 ou 2, dans laquelle la substance amère ou astringente inclut un extrait de houblon.

4. Composition pour une administration orale selon l'une quelconque des revendications précédentes, dans laquelle la substance amère ou astringente inclut un acide iso-α.

5. Composition pour une administration orale selon l'une quelconque des revendications précédentes, dans laquelle la substance amère ou astringente inclut une combinaison d'un acide iso-α et d'un acide-α.

6. Composition pour une administration orale selon l'une quelconque des revendications précédentes, dans laquelle le dispersant est une macromolécule soluble dans l'eau et/ou un tensioactif.

7. Composition pour une administration orale selon la revendication 6, dans laquelle le dispersant est une macromolécule soluble dans l'eau sélectionnée parmi la gélatine, la gomme arabique, de l'amidon modifié, le pullulane et un dérivé de cellulose.

8. Composition pour une administration orale selon l'une quelconque des revendications précédentes, contenant 0,01 à 100 moles d'un sel de métal polyvalent par mole de substance amère ou astringente.

9. Composition pour une administration orale selon l'une quelconque des revendications précédentes, dans laquelle la teneur en dispersant est de 0,1 fois à 100 fois la teneur en substance amère ou astringente en termes de rapport massique.

10. Composition pour une administration orale selon l'une quelconque des revendications précédentes, qui est sous la forme d'un liquide de dispersion incluant des particules dispersées, et les particules dispersées incluent le matériau insolubilisé dans l'eau.

11. Composition pour une administration orale selon la revendication 10, dans laquelle le diamètre de particule moyen des particules dispersées est 300 nm ou moins.

12. Procédé de réduction de l'amertume ou de l'astringence d'une composition qui inclut une substance amère ou astringente, le procédé incluant combiner la substance amère ou astringente avec un dispersant et un sel de fer trivalent.
